Europäisches Patentamt

(19) European Patent Office

Office européen des brevets

(11) Veröffentlichungsnummer: **0 139 985**
**B1**

(12) **EUROPÄISCHE PATENTSCHRIFT**

(45) Veröffentlichungstag der Patentschrift:
16.12.87

(51) Int. Cl.⁴: **C 12 Q 1/52**

(21) Anmeldenummer: 84110018.3

(22) Anmeldetag: 22.08.84

(54) **Verfahren und Reagenz zur Bestimmung von Transaminasen.**

(30) Priorität: 22.08.83 DE 3330246

(43) Veröffentlichungstag der Anmeldung:
08.05.85 Patentblatt 85/19

(45) Bekanntmachung des Hinweises auf die Patenterteilung:
16.12.87 Patentblatt 87/51

(84) Benannte Vertragsstaaten:
AT BE CH DE FR GB IT LI LU NL SE

(56) Entgegenhaltungen:
DE-A-3 026 854
US-A-3 527 332

(73) Patentinhaber: Boehringer Mannheim GmbH,
Sandhofer Strasse 112- 132, D-6800 Mannheim
Waldhof (DE)

(72) Erfinder: Möller, Gerald, Dr., Waxensteinstrasse
17, D-8132 Tutzing (DE)

(74) Vertreter: Weickmann, Heinrich, Dipl.- Ing.,
Patentanwälte Dipl.- Ing. H.Weickmann Dipl.-
Phys.Dr. K.Fincke Dipl.- Ing. F.A.Weickmann
Dipl.- Chem. B. Huber Dr.- Ing. H. Liska Dipl.-
Phys.Dr. J. Prechtel Postfach 860820, D-8000
München 86 (DE)

EP 0 139 985 B1

## Beschreibung

Menschliches Gewebe enthält die Transaminasen Glutamatpyruvat-Transaminase (GPT) EC 2.6.1.2 und Glutamat-Oxalacetat-Transaminase (GOT) EC 2.6.1.1 in Muskeln, Serum und Organen. Die Messung des GPT-Gehaltes insbesondere im Serum, stellt einen wichtigen klinischen Parameter zur Unterscheidung von Leber- und Herzerkrankungen dar.

Die GPT kommt fast ausschließlich in der Leber vor, wo sie nur im Zytoplasma der Parenchymzellen vorhanden ist, während die GOT (Glutamat-Oxalacetat-Transaminase) zu je etwa 50 % im Zytoplasma und in den Mitochondrien vorliegt. Aus dieser Lokalisation der beiden Transaminasen ergeben sich wertvolle diagnostische Hinweise bei den verschiedenen Lebererkrankungen. Höhere GOT-Werte sprechen für eine schwere Leberparenchymzellschädigung.

Bei akuter Hepatitis sind stets GOT und GPT im Serum erhöht, auch bei anikterischem Verlauf. Oft kann man den Enzymaktivitätsanstieg schon vor dem Erscheinen des Ikterus nachweisen.

Bei chronischer Hepatitis und Cirrhose sind GOT und GPT niedriger als bei der Virushepatitis. Ihre Bestimmung ist bei den chronischen Leberentzündungen vor allem zur Überwachung des Krankheitsverlaufes und der Therapie wertvoll.

Die Bestimmung der GOT wird daneben auch zur Diagnostik von Erkrankungen des Herzmuskels eingesetzt.

Ein übliches und viel verwendetes Verfahren zur Bestimmung von GPT bzw. GOT benutzt die Transaminierung von α-Ketoglutarsäure mit Alanin bzw. Aspartat, die durch diese Enzyme katalysiert wird und zur Bildung von Glutamatsäure und Pyruvat bzw. Oxalacetat führt. Pyruvat bzw. Oxalacetat wird durch Reduktion mit NADH in Gegenwart von Lactatdehydrogenase (LDH) und gegebenenfalls MDH in Lactat bzw. Malat und NAD+ umgesetzt, wobei letzteres nach bekannten Methoden entweder direkt im UV oder mit Hilfe einer nachgeschalteten Farbreaktion gemessen wird.

Ein wesentlicher Nachteil dieses Verfahrens besteht nun darin, daß LDH selbst eine Unspezifität (Nebenaktivität) aufweist, die zu einer Umsetzung von α-Ketosäuren, insbesondere von α-Keto-glutarsäure unter Bildung von α-Hydroxyglutarsäure führt und als α-Hydroxyglutarat-Dehydrogenase-Aktivität bezeichnet werden kann.

Diese Unspezifität hat außerdem zur Folge, daß ein entsprechendes Reagenz zur Durchführung dieses bekannten Verfahrens nur eine ungenügende Stabilität aufweist, da bei der Bildung von α-Hydroxyglutarat NADH zu NAD oxidiert wird. Dies verursacht eine Schleichreaktion.

Der Erfindung liegt die Aufgabe zugrunde, dieses Problem zu lösen.

Erfindungsgemäß gelingt dies bei einem Verfahren zur Bestimmung der Transaminasen durch Umsetzung einer entsprechenden Aminosäure mit α-Ketoglutarsäure zu Glutamatsäure und der der eingesetzten Aminosäure entsprechenden α-Ketosäure und Messung der gebildeten α-Ketosäure durch Reduktion mit NADH in-Gegenwart von Lactatdehydrogenase (LDH) und gegebenenfalls Malatdehydrogenase (MDH) unter Bildung von NAD+ und α-Oxisäure, welches dadurch gekennzeichnet ist, daß man D-LDH eines Milchsäurebakteriums verwendet.

Die Erfindung beruht auf der überraschenden Entdeckung, daß D-LDH von Milchsäurebakterien auch in Gegenwart von NADH α-Oxoglutarsäure nicht reduziert. Ersetzt man daher die bisher üblicherweise verwendete LDH aus Schweineherz in dem oben angegebenen Verfahren erfindungsgemäß durch D-LDH eines Milchsäurebakteriums, so tritt die störende Schleichreaktion nicht mehr auf und man erhält ein hinsichtlich seiner Lagerstabilität wesentlich verbessertes Reagenz.

Die vorteilhaften Ergebnisse gemäß der Erfindung erhält man mit jeder D-LDH aus Milchsäurebakterien. D-LDH bildende Milchsäurebakterien gehören im wesentlichen den Gattungen Lactobacillus, Pediococcus und Leuconostoc an (Antonie van Leeuwenhoek 49, (1983) 210). Soweit Milchsäurebakterien L-LDH bilden, ist diese nicht geeignet. Soweit ein Stamm sowohl D-LDH als auch L-LDH bildet, muß eine Trennung dieser beiden Enzyme durchgeführt werden. Bevorzugt werden daher D-LDH-Präparate aus Stämmen, die lediglich D-LDH enthalten. Besonders gute Ergebnisse ergibt die LDH aus Lactobacillus leichmannii, DSM 2699, bei der es sich um eine D-LDH handelt. Weitere bevorzugte Stämme sind Lactobacillus lactis, DSM 20072, Lactobacillus plantarum, DSM 20174, Leuconostoc mesenteroides DSM 20193 oder Pediococcus pentosaceus DSM 20280.

Wird das erfindungsgemäße Verfahren zur Bestimmung der Glutamat-Pyruvat-Transaminase (GPT) eingesetzt, so verwendet man als Aminosäure Alanin, welches zu Pyruvat desaminiert und dann zu Lactat reduziert wird. Handelt es sich um die Bestimmung der Glutamat-Oxalacetat-Transaminase (GOT) so wird als aminosäure Aspartat eingesetzt, welches zu Oxalacetat desaminiert und dann zu Malat reduziert wird.

Ein erfindungsgemäßes Reagenz zur Bestimmung von Transaminasen, enthaltend LDH, α-Oxoglutarat, die entsprechende Aminosäure, NADH, Puffer pH 6,5 bis 8,5 und gegebenenfalls MDH, zeichnet sich daher dadurch aus, daß es D-LDH eines Milchsäurebakteriums enthält.

Im Falle der GPT-Bestimmung enthält das Reagenz als Aminosäure Alanin. Im Falle der GOT-Bestimmung enthält das Reagenz als Aminosäure Aspartat und außerdem Malatdehydrogenase (MDH).

In einer bevorzugten Ausführungsform enthält das erfindungsgemäße Reagenz zur Bestimmung von GPT

250 bis 20000 U/l D-LDH aus Milchsäurebakterien

2,5 bis 100 mMol/l α-Oxoglutarat,
50 bis 1000 mMol/l Alanin,
0,01 bis 0,25 mMol/l NADH und
10 bis 500 mMol/l Puffer pH 6,5 bis 8,5.

In einer weiteren bevorzugten Ausführungsform enthält das erfindungsgemäße Reagenz zur Bestimmung von GOT

250 bis 20000 U/l D-LDH aus Milchsäurebakterien

2,5 bis 100 mMol/l α-Oxoglutarat
20 bis 1000 mMol/l Aspartat
0,01 bis 0,25 mMol/l NADH
100 bis 20000 U/l MDH und
10 bis 500 mMol/l Puffer pH 6,5 bis 8,5.

Im Rahmen der Erfindung beträgt die besonders bevorzugte Endkonzentration der einzelnen Bestandteile des GPT-Puffers im Test LDH: 1000 bis 1400 U/l, Alanin 600 bis 800 mMol/l, NADH 0,10 bis 0,20 mMol/l, α-Oxoglutarat 10 bis 25 mMol/l und Puffer 50 bis 100 mMol/l pH 7,3 bis 7,5.

Die entsprechenden Werte für die Konzentration im Test beim GOT-Reagenz sind 1000 bis 1400 U/l LDH, 500 bis 1000 U/l MDH, 0,10 bis 0,20 mMol/l NADH, 150 bis 250 mMol/l Aspartat und 10 bis 20 mMol/l α-Oxoglutarat und Puffer 50 bis 100 mMol/l pH 7,3 bis 7,5.

Als Puffer kommen die im angegebenen pH-Bereich wirksamen Puffersubstanzen in betracht. Bevorzugt werden Trispuffer, Trapuffer (Triethanolamin) und Phosphatpuffer.

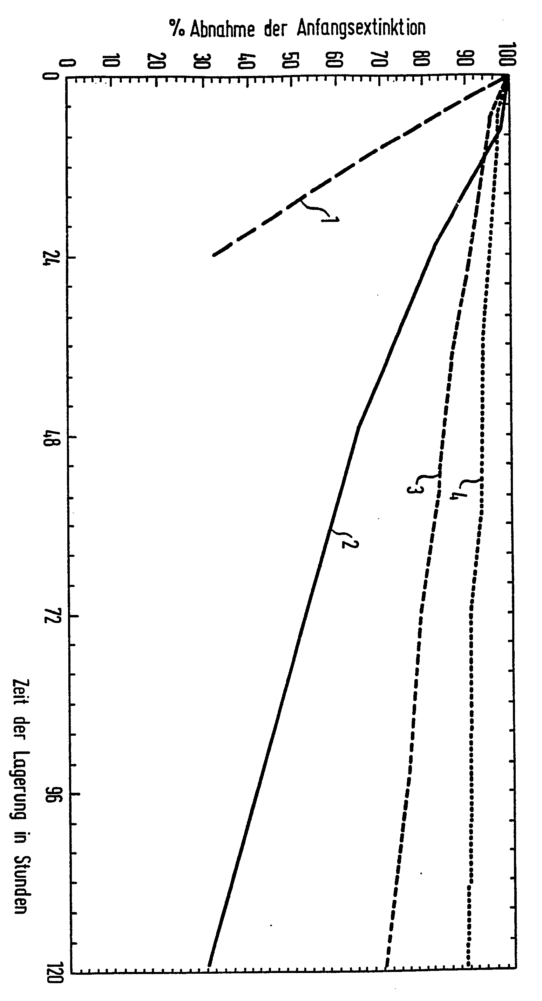

Der erfindungsgemäß erzielte Effekt ist in der Figur der beigefügten Zeichnung dargestellt. In dieser wird die prozentuale Abnahme der Anfangsextinktion gegen die Lagerungszeit in Stunden für ein bekanntes Verfahren bzw. Reagenz im Vergleich zu einem erfindungsgemäßen Verfahren bzw. Reagenz angegeben. Für die Bestimmung der in der Figur wiedergegebenen Werte wurde ein im Handel unter der Bezeichnung "Monotest® a ALAT/ALT/GPT" erhältliches Reagenz zur Bestimmung von GPT der Boehringer Mannheim GmbH verglichen mit einem erfindungsgemäßen Reagenz. Die Konzentration im Test war wie folgt:

1200 U/l LDH,
0,180 mMol/l NADH,
15 mMol/l α-Oxoglutarat,
500 mMol/l Alanin,
100 mMol/l Trispuffer pH 7,5.

Das bekannte Reagenz enthielt LDH aus Schweineherz; das erfindungsgemäße Reagenz enthielt D-LDH aus Lactobacillus leichmannii DSM 2699.

In der Figur stellen dar:
Kurve 1 das handelsübliche Reagenz bei 25°C (Wasserbad),
Kurve 2 das handelsübliche Reagenz bei +4°C (Eisbad),
Kurve 3 das erfindungsgemäße Reagenz bei 25°C (Wasserbad),
Kurve 4 das erfindungsgemäße Reagenz bei +4°C (Eisbad).

Man erkennt daraus, daß bei 25°C beim bekannten Reagenz nach etwa 34 Stunden das gesamte NADH verschwunden ist, während beim erfindungsgemäßen Reagenz noch nach 120 Stunden ca. 71 % der Anfangsextinktion erhalten werden. Die entsprechenden Werte bei 4°C und 120 Stunden Lagerungszeit sind für das bekannte Reagenz 30 % Restextinktion, für das erfindungsgemäße Reagenz 90 %. Analoge Ergebnisse wie bei Schweineherz LDH werden erhalten, wenn anstelle von LDH aus Schweineherz L-LDH aus L. casei, L. xylosus, B. Stearothermophilus oder L. plantarum eingesetzt wird.

Identische Kurven werden auch erhalten, wenn man anstelle des oben angegebenen handelsüblichen Reagenz für die GPT-Bestimmung ein entsprechendes handelsübliches Reagenz für die GOT-Bestimmung verwendet, welches unter der Bezeichnung "Monotest® a ASAT/AST/GOT" erhältlich ist. Die Konzentration im Test war hierbei wie folgt:

600 U/l LDH,
420 U/l MDH,
0,180 mMol/l NADH,
12 mMol/l α-Oxoglutarat,
240 mMol/l Aspartat,
80 mMol/l Trispuffer pH 7,8.

Bei diesem Reagenz ist ein Gehalt an LDH erforderlich, da jede Probe endogenes Pyruvat und in der Regel auch endogene LDH enthält. Dies führt zu einer Schleichreaktion durch langsamen Abbau von Pyruvat, der durch Zusatz von überschüssiger, erfindungsgemäß einzusetzender LDH innerhalb kurzer Zeit abläuft.

Diese "Nebenreaktion" tritt naturgemäß auch bei der GPT-Bestimmung in gleicher Weise auf, wird hier jedoch von dem größeren Problem der α-Hydroxyglutaratdehydrogenase-Aktivität überlagert. Durch die Erfindung werden jedoch beide Probleme gleichzeitig gelöst.

Daraus ergibt sich, daß erfindungsgemäß eine Vervielfachung der Lagerungsbeständigkeit erzielt wird.

Ein für die Erfindung geeignetes Enzympräparat aus Lactobacillus leichmannii DSM 2699 kann nach den bekannten Verfahren zur Gewinnung von mikrobieller LDH hergestellt werden, beispielsweise nach dem in J. of General Microbiology (1970) 62, 243 beschriebenen Verfahren. Dieses Verfahren wird im Prinzip wie folgt durchgeführt:

Zellaufschluß von in 0,05 M Kaliumphosphat-Puffer pH 7 suspendiertem Lactobacillus mittels Ultraschall. Zentrifugation und Verwerfen der Zelltrümmer.

Der erhaltene Mikroorganismus-Rohextrakt wird mit Essigsäure auf pH 5,5 gebracht, mit Protaminsulfat auf 0,3 % G/V versetzt und der Niederschlag abzentrifugiert. Der Überstand wird auf pH 7 eingestellt und die mit Ammoniumsulfat zwischen 50 und 85 % Sättigung ausfallende Fraktion gewonnen. Diese Fraktion wird in Puffer pH 7 über einen schwachen Anionenaustauscher (DEAE-Sephadex® A 50) chromatographiert und mit einem NaCl-Gradienten eluiert.

Man erhält mit ca. 25 %-iger Ausbeute ein LDH-Präparat, welches gegenüber dem Rohextrakt um den Faktor 30 oder mehr gereinigt ist. Dieses Verfahren eignet sich auch für die anderen Stämme der Gattung Lactobacillus und die Gattung Leuconostoc, z. B. für L. lactis DSM 20072, L. plantarum DSM 20174 und Leuconostoc mesenteroides DSM 20193. Bei Pediococcus-Stämmen wird das Verfahren gemäß J. of Bacteriology, Vol. 121, p. 602 angewendet.

Die folgenden Beispiele erläutern die Erfindung weiter.

**Beispiel 1**

Bestimmung der GPT-Aktivität (Version mit Phosphatpuffer)
Probenmaterial: Serum, Heparin- oder EDTA-Plasma
Reagenzien: (Endkonzentrationen im Test)

Phosphatpuffer 80 mMol/l pH 7,4
| | |
|---|---|
| L-Alanin | 800 mMol/l |
| LDH | ⩽ 1200 U/l |
| NADH | 0,18 mMol/l |
| α-Ketoglutarat | 18,0 mMol/l |

Bestimmungsansatz
Wellenlänge: Hg 385 nm, 340 nm oder Hg 334 nm
Küvette: 1 cm Schichtdicke
Meptemperatur: 25° C
Messung gegen Luft (Extinktionsabnahme)
Bei Photometern mit Analoganzeige Anfangsextinktionen über 0,500 mit Stufenschaltung kompensieren.
> r
In eine Küvette pipettieren:
| | |
|---|---|
| Reaktionsgemisch (25° C) | 2,5 ml |
| Probe | 0,5 ml |

mischen. Nach ca. 1 Minute Extinktion ablesen und gleichzeitig Stoppuhr starten. Nach genau weiteren 1, 2 und 3 Minuten Ablesung wiederholen.

Bei Extinktionsdifferenzen pro Minute (ΔE/min) von 0,06 bis 0,08 (Hg 365) bzw. 0,11 bis 0,18 (Hg 334 und 340 nm) werden nur die Messungen der ersten zwei Minuten berücksichtigt (1 Minute inkubieren und 2 Minuten messen).
Aus den Extinktionsdifferenze pro Minute (ΔE/min) Mittelwert bilden und diesen in die Berechnungen einsetzen.
Berechnung
Die Aktivität der GPT in der Probe berechnen nach:
$$U/l \ (25°C) = 1785 \times \Delta E_{365} \ nm/Min$$
$$U/l \ (25°C) = 952 \times \Delta E_{340} \ nm/Min$$
$$U/l \ (25°C) = 971 \times \Delta E_{334} \ nm/Min$$

**Beispiel 2**

Bestimmung der GOT-Aktivität (Version mit TRIS-Puffer)
Probenmaterial: Serum, Heparin- oder EDTA-Plasma
Reagenzien: (Endkonzentrationen im Test)

| | |
|---|---|
| Tris-Puffer | 100 mMol/l pH 7,5 |
| NADH | 0,18 mMol/l |
| MDH | 800 U/l |
| LDH | 1200 U/l |
| α-Ketoglutarat | 12 mMol/l |
| L-Aspartat | 200 mMol/l |

Bestimmungsansatz:
Wellenlänge: Hg 365 nm, 340 nm oder 334 nm
Küvette: 1 cm Schichtdicke
Meßtemperatur: 25° C
Messung gegen Luft (Extinktionsabnahme)

| | |
|---|---|
| Reagenzlösung (25° C) | 2,5 ml |
| Probe | 0,5 ml |

mischen, Lösung in Küvette gießen. Nach ca. 1 Minute Extinktion ablesen und gleichzeitig Stoppuhr starten. Nach genau weiteren 1, 2 und 3 Minuten Ablesung wieder holen.

Aus den Extinktionsdifferenzen pro Minute (ΔE/min) Mittelwert bilden und diesen in die Berechnung einsetzen.
Berechnung
Die Aktivität der GOT in der Probe aus der Tabelle entnehmen oder berechnen nach
$$U/l \ (25°C) = 2059 \times \Delta E_{365} \ nm/min$$
$$U/l \ (25°C) = 1111 \times \Delta E_{340} \ nm/min$$
$$U/l \ (25°C) = 1133 \times \Delta E_{334} \ nm/min.$$

**Patentansprüche**

1. Verfahren zur Bestimmung der Transaminasen durch Umsetzung einer entsprechenden Aminosäure mit α-Ketoglutarsäure zu Glutarsäure und der der eingesetzten Aminosäure entsprechenden α-Ketosäure und Messung der gebildeten α-Ketosäure durch Reduktion mit NADH in Gegenwart von Lactatdehydrogenase (LDH) und gegebenenfalls Malatdehydrogenase (MDH) unter Bildung von NAD+ und α-Oxisaure, dadurch gekennzeichnet, daß man D-LDH eines Milchsäurebacteriums verwendet.

2. Verfahren nach Anspruch 1, dadurch gekennzeichnet, daß man D-LDH aus Mikroorganismen der Gattung Lactobacillus Pediococcus oder/und Leuconnostoc verwendet.

3. Verfahren nach Anspruch 1 oder 2, dadurch gekennzeichnet, daß -man Glutamat-Pyruvat-Transaminase (GPT) bestimmt und als Aminosäure Alanin einsetzt, welches zu Pyruvat desaminiert und zu Lactat reduziert wird.

4. Verfahren nach Anspruch 1 oder 2, dadurch gekennzeichnet, daß man Glutamat-Oxalacetat-Transaminase (GOT) bestimmt und als Aminosäure Aspartat einsetzt, welches zu Oxalacetat desaminiert und zu Malat reduziert wird.

5. Verfahren nach einem der Ansprüche 2 bis 4, dadurch gekennzeichnet, daß man D-LDH aus Lactobacillus leichmannii DSM 2699, Lactobacillus lactis DSM 20072, Lactobacillus plantarum DSM 20174, Leuconostoc mesenteroides DSM 20193 oder Pediococcus pentosaceus DSM 20280 verwendet.

6. Reagenz zur Bestimmung der Transaminasen, enthaltend LDH, α-Oxoglutarat, die entsprechende Aminosäure, NADH, Puffer pH 6,5 bis 8,5 und gegebenenfalls MDH, dadurch gekennzeichnet, daß es D-LDH eines Milchsäurebacteriums enthält.

7. Reagenz nach Anspruch 6 zur Bestimmung von GPT, dadurch gekennzeichnet, daß es als Aminosäure Alanin enthält.

8. Reagenz nach Anspruch 6 zur Bestimmung von GOT, dadurch gekennzeichnet, daß es als Aminosäure Aspartat enthält.

9. Reagenz nach Anspruch 7, enthaltend 250 bis 20000 U/l D-LDH aus Milchsäurebakterien,
2,5 bis 100 mMol/1 α-Oxoglutarat,
50 bis 1000 mMol/l Alanin,
0,01 bis 0,25 mMol/l NADH und
10 bis 500 mMol/l Puffer pH 6,5 bis 8,5.

10. Reagenz nach Anspruch 8, enthaltend 250 bis 20000 U/l D-LDH aus Milchsäurebakterien
2,5 bis 100 mMol/l a-Oxoglutarat
20 bis 1000 mMol/l Aspartat
0,01 bis 0,25 mMol/l NADH
100 bis 20000 U/l MDH und
10 bis 500 mMol/l Puffer pH 6,5 bis 8,5.

11. Reagenz nach einem der Ansprüche 6 bis 10, dadurch gekennzeichnet, daß es D-LDH aus einem Mikroorganismus der Gattung Lactobacillus Pediococcus oder/und Leuconostoc enthält.

12. Reagenz nach Anspruch 11, dadurch gekennzeichnet daß es D-LDH aus Lactobacillus leichmanii DSM 2699, Lactobacillus lactis DSM 20072, Lactobacillus plantarum DSM 20174, Leuconostoc mesenteroides DSM 20193 oder Pediococcus pentosaceus DSM 20280 verwendet.

## Claims

1. Process for the determination of transaminases by reaction of an appropriate amino acid with α-ketoglutaric acid to glutaric acid and to the α-ketoacid corresponding to the amino acid used and measurement of the α-keto acid formed by reduction with NADH in the presence of lactate dehydrogenase (LDH) and possibly of malate dehydrogenase (MDH) with formation of NAD+ and α-oxy acid, characterised in that one uses D-LDH of a lactic acid bacterium.

2. Process according to claim 1, characterised in that one uses D-LDH from micro-organisms of the species Lactobacillus pediococcus and/or Leuconostoc.

3. Process according to claim 1 or 2, characterised in that one determines glutamate-pyruvate-transaminase (GPT) and uses alanine as amino acid which is desaminated to pyruvate and reduced to lactate.

4. Process according to claim 1 or 2, characterised in that one determines glutamate-oxalacetate-transaminase (GOT) and uses aspartate as amino acid which is desaminated to oxalacetate and reduced to malate.

5. Process according to one of claims 2 to 4, characterised in that one uses D-LDH from Lactobacillus leichmannii DSM 2699, Lactobacillus lactis DSM 20072, Lactobacillus plantarum DSM 20174, Leuconostoc mesenteroides DSM 20193 or Pediococcus pentosaceus DSM 20280.

6. Reagent for the determination of transaminases, containing LDH, α-oxoglutarate, the corresponding amino acid, NADH, buffer pH 6.5 to 8.5 and possibly MDH, characterised in that it contains D-LDH of a lactic acid bacterium.

7. Reagent according to claim 6 for the determination of GPT, characterised in that it contains alanine as amino acid.

8. Reagent according to claim 6 for the determination of GOT, characterised in that it contains aspartate as amino acid.

9. Reagent according to claim 7, containing 250 to 20000 U/l. D-LDH from lactic acid bacteria,
2.5 to 100 mMole/l. α-oxoglutarate,
50 to 1000 mMole/l. alanine,
0.01 to 0.25 mMole/l. NADH and
10 to 500 mMole/l. buffer pH 6.5 to 8.5.

10. Reagent according to claim 8, containing 250 to 20000.U/l. D-LDH from lactic acid bacteria,
2.5 to 100 mMole/l. α-oxoflutarate,
20 to 1000 mMole/l. aspartate,
0.01 to 0.25 mMole/l. NADH,
100 to 2000 U/l. MDH and
10 to 500 mMole/l. buffer pH 6.5 to 8.5.

11. Reagent according to one of claims 6 to 10, characterised in that it contains D-LDH from a microorganism of the species Lactobacillus pediococcus and/or Leuconostoc.

12. Reagent according to claim 11, characterised in that it uses D-LDH from Lactobacillus leichmannii DSM 2699, Lactobacillus lactis DSM 20072, Lactobacillus plantarum DSM 20174, Leuconostoc mesenteroides DSM 20193 or Pediococcus pentosaceus DSM 20280.

## Revendications

1. Procédé de détermination des transaminases par réaction d'un amino-acide approprié avec

l'acide α-cétoglutarique pour donner l'acide glutarique et l'α-cétoacide correspondant à l'amino-acide utilisé et mesure de l'α-cétoacide formé par réduction avec le NADH en presence de lactate-deshydrogenase (LDH) et le cas échéant de malate-deshydrogénase (MDH) avec formation de NAD+ et d'α-oxyacide, caractérisé en ce qu'on utilise une D-LDH d'une bactérie lactique.

2. Procédé suivant la revendication 1, caractérisé en ce qu'on utilise une D-LDH de microorganismes du genre Lactobacillus Pediococcus et/ou Leuconostoc.

3. Procédé suivant la revendication 1 ou 2, caractérisé en ce qu'on détermine la glutamate-pyruvate-transaminase (GPT) et en ce qu'on utilise comme aminoacide l'alanine, qui est désaminée en pyruvate et réduite en lactate.

4. Procédé suivant la revendication 1 ou 2, caractérisé en ce qu'on détermine la glutamate-oxalacétate-transaminase (GOT) et en ce qu'on utilise comme amino-acide l'aspartate, qui est désaminé en oxalacétate et réduit en malate.

5. Procédé suivant l'une des revendications 2 à 4, caractérisé en ce qu'on utilise de la D-LDH de Lactobacillus leichmannii DSM 2699, Lactobacillus lactis DSM 20072, Lactobacillus plantarum DSM 20174, Leuconostoc mesenteroides DSM 20193 ou Pediococcus pentosaceus DSM 20280.

6. Réactif pour la détermination des transaminases, contenant de la LDH, de l'α-oxoglutarate, l'aminoacide correspondant, du NADH, un tampon à pH 6,5 à 8,5 et le cas échéant de la MDH, caractérisé en ce qu'il contient de la D-LDH d'une bactérie lactique.

7. Réactif suivant la revendication 6, pour la détermination de la GPT, caractérisé en ce qu'il contient comme amino-acide de l'alanine.

8. Réactif suivant la revendication 6, pour la détermination de la GOT, caractérisé en ce qu'il contient comme amino-acide de l'aspartate.

9. Réactif suivant la revendication 7, contenant 250 à 20000 U/l de D-LDH de bactéries lactiques,
2,5 à 100 mmoles/l d'α-oxoglutarate,
50 à 1000 mmoles/l d'alanine,
0,01 à 0,25 mmole/l de NADH et
10 à 500 mmoles/l de tampon à pH 6,5 à 8,5.

10. Réactif selon la revendication 8, contenant 250 à 20000 U/l de D-LDH de bactéries lactiques,
2,5 à 100 mmoles/l d'α-oxoglutarate,
20 à 1000 mmoles/l d'aspartate,
0,01 à 0,25 mmole/l de NADH,
100 à 20000 U/l de MDH et
10 à 500 mmoles/l de tampon à pH 6,5 à 8,5.

11. Réactif suivant l'une des revendications 6 à 10, caractérisé en ce qu'il contient de la D-LDH provenant d'un microorganisme du genre Lactobacillus, Pediococcus et/ou Leuconostoc.

12. Réactif suivant la revendication 11, caractérisé en ce qu'il utilise de la D-LDH de Lactobacillus leichmannii DSM 2699. Lactobacillus lactis DSM 20072, Lactobacillus plantarum DSM 20174, Leuconostoc mesenteroides DSM 20193 ou Pediococcus pentosaceus DSM 20280.